# EUROPEAN PATENT SPECIFICATION

(11) **EP 1 925 312 B1**
(45) Date of publication and mention of the grant of the patent: **03.08.2011**
(21) Application number: 07022830.9
(22) Date of filing: 26.11.2007
(51) Int. Cl.: A61K 36/484, A61P 3/10

(54) **Fermented licorice root for treatment of complications of diabetes mellitus**
Fermentierte Süssholzwurzel zur Behandlung von Komplikationen bei Diabetes mellitus
Racine de réglisse fermentée pour le traitement des complications des diabètes mellitus

(30) Priority: 26.11.2006 EG 20060609
(43) Date of publication of application: 28.05.2008
(73) Proprietor: Massoud, Ahmed Mohamed Ali, Dr., Kairo (EG)
(72) Inventor: Massoud, Ahmed Mohamed Ali, Dr., Kairo (EG)
(74) Representative: Walther, Walther & Hinz GbR

(56) References cited:
- STEPANOVA: "Study of the fermentation process of glycyrrhiza glabra l. herb" FARMATSIYA, vol. 34, 1985, pages 20-22, XP008089646 Moscow (in Russian)
- FUHRMAN ET AL: "Antiatherosclerotic effects of licorice extract supplementation on hypercholesterolemic patients: Increased resistance of LDL to atherogenic modifications, reduced plasma lipid levels, and decreased systolic blood pressure" NUTRITION, vol. 18, 2002, pages 268-273, XP002473201
- ISBRUCKER ET AL: "Risk and safety assessment on the consumption of Licorice root (Glycyrrhiza sp.), its extract and powder as a food ingredient, with emphasis on the pharmacology and toxicology of glycyrrhizin" REGULATORY TOXICOLOGY AND PHARMACOLOGY, vol. 46, December 2006 (2006-12), pages 167-192, XP005740964
- DATABASE BIOSIS [Online] BIOSCIENCES INFORMATION SERVICE, PHILADELPHIA, PA, US; October 2007 (2007-10), CHEN YONG-QIANG ET AL: "Microbial transformation of liquorice to enhance the pharmaceutical effects" XP002473284 Database accession no. PREV200800141925 & SICHUAN DAXUE XUEBAO (ZIRAN KEXUEBAN), vol. 44, no. 5, October 2007 (2007-10), pages 1147-1150, ISSN: 0490-6756

## Description

### Prior art

The total number of diabetics allover the world is more than 300 millions (5-6% of world population). There are two types of diabetes mellitus:
1. Type one diabetes mellitus which is also called (insulin dependent diabetes mellitus). It represents about 10% of total diabetic cases and is treated by insulin only.
2. Type two diabetes mellitus which is also called (non insulin dependent diabetes mellitus), or adult type. It represents about 90% of total diabetic cases. It is treated by oral antidiabetics which aim at reducing blood sugar level. They can be classified into 3 main groups :
   A. Sulphonyl ureas.
   B. Metformin.
   C. Insulin sensitizers which activate insulin receptors in body cells. This group of drugs is called thiazolidine diones which includes rosiglitazone as avandia, pioglitazone as glustin.
3. Stepanova: "Study of the fermentation in process of glycyrrhiza glabra I. herb" Farmatsiya, vol. 34, 1985, pages 20-22 XP008089646 discloses a fermentation process to influence the yield of saponins from glycyrrhiza glabra I. herb. The qualitative yield of triterpene saponinsis increased by more than 50% as compared to control, inactivated and unfermented samples.

### Disadvantages in previous art

The present problem for diabetic patients is the persistence of complications which increase by time inspite of treatment, as all drugs used today aim at lowering the blood sugar level through:
A. Injection of insulin.
B. Stimulation of insulin secretion by beta cells of pancreas (action of sulphonyly ureas).
C. Lowering blood glucose through direct stimulation of glycolysis, slowing of absorption of glucose in intestine and reduction of gluconeogenesis in liver (action of biguanides).
D. Insulin sensitizer (action of thiazolidine diones which include pioglitazone as glustin and rosiglitazone as avandia). But the main problem of diabetes - besides hyperglycemia - is the severe and multiple complications of the disease that increase and become more severe by time even in case of success in blood sugar control. The most important complications of diabetes are:
   1. Cataract and retinopathy that may cause diminution of vision.
   2. Nephropathy that may end in renal failure. Diabetes mellitus is one of the most important causes of renal failure.
   3. Peripheral neuritis which may cause pain, numbness and loss of sensation. This is a serious complication as it may end in foot gangrene in case of unnoticed trauma because of the present peripheral arteritis and poor blood supply of foot which are common in diabetes mellitus
   4. Peripheral arteritis.
   5. Cerebral vascular disease.
   6. Hypertension.
   7. Coronary insufficiency.
   8. Rise in serum uric acid.
   9. Atherosclerosis and dyslipidemia.
   10. Hyper coagulability.

All complications of diabetes mellitus persist and become more intensified by time inspite of the regular follow up and treatment of hyperglycemia, all available treatment for these complications are only symptomatic. This is a big problem which necessitates searching for the etiology of these complications and hence the etiological treatment, other than the symptomatic treatment used nowadays.

### Advantages of the new drug

This drug aims at treatment of complications of diabetes mellitus which are very common. They persist and become more intensified by time in spite of control of blood sugar levels. These complications represent the most serious problem of diabetes mellitus. To search for a drug that can prevent these complications, we had to study the main role of enzymes in metabolic reactions allover the body because there is a strict relationship between enzymatic deficiency and the metabolic disturbances which cause all diabetic complications.

This was confirmed by the following facts:

### First:

All metabolic activities in the body are carried out in the form of chain of enzymatic reactions. Deficiency of specific metabolic enzymes causes abnormal metabolism (6-22). Researches concerning diabetic complications showed that these complications start by disturbance of glucose metabolism inside the cell and tissues of diabetic patient. Normally glucose catabolism is the major source energy for cellular processes. In diabetic patient, disturbance of glucose metabolism in cells will be accompanied and complicated by the following:
A. Liver glycogen is depleted (5).
B. Protein becomes the only important source of energy. This causes great increase of protein catabolism, decrease of protein synthesis in muscles which end in muscle weakness.
C. Great increase of fat catabolism which causes several complications caused by dyslipidemia. Thus, the main problem that causes all these complications is the disturbance of glucose metabolism in cells which is carried out - as mentioned - in the form of enzymatic reactions -by specific metabolic enzymes.

### Second:

Recent researches in hepatology showed that complications of diabetes mellitus are associated with mitochondrial dysfunction in liver cells. Mitochondria in the liver cells, are the site of energy production which enables these cells to carry out their normal metabolic functions. Mitochondria also represent the cross roads for the three major metabolic pathways, carbohydrate, lipids and protein. Thus, they render the liver capable of directing one metabolic pathway in favor of the others in order to attain the metabolic balance required. The relationship between the degree of mitochondrial disturbance and the degree of diabetic complications is of great importance (14, 15, 16).

### Third:

Recently it was discovered that the role of what is called (digestive enzymes) is not digestion only, but they are absorbed in the intestine, they are carried to the liver where they have an important role in metabolic reactions in the liver and all body tissues (6, 8, 9, 17, 18, 19)
It was proved also that pancreatic amylase - besides its role in digestion - is absorbed in the intestine and reaches the liver. This circulation was called entero - hepatic circulation and entero - pancreatic circulation (6) Besides, researches showed that deficiency in food enzymes leads to hypertrophy of pancreas and salivary glands to compensate for digestive enzymes, and this hypertrophy can be relieved by oral supply of sufficient amounts of these enzymes. If deficiency of these enzymes in diet for a longer time it may cause deficiency of metabolic activities all over the body (6).

Accordingly, we searched for a drug that can treat the presenting complications of diabetes mellitus gradually and prevent further complications and deterioration by supplying the patient with sufficient amounts of enzymes which are necessary for the normal enzymatic activity to regain the normal metabolism allover the body. As for the enzymes to be used we gave the priority to amylase enzyme, as it is the enzyme concerned with carbohydrate metabolism. Also liver cells identify this enzyme and receive it from the blood through the specific receptors called mannose receptors which are found on liver cell surfaces (13)
Besides, amylase enzyme has high affinity to liver glycogen (1). All above mentioned facts confirm the important role of amylase in carbohydrate metabolism and in liver metabolism which affects all body functions.

Also we searched for a common plant which is rich in enzymes. We tried to avoid synthetic drugs or strange substances which may not be easily absorbed . It is well known now that nutrients are absorbed through the intestinal enterocytes by active transport which moves the nutrients against chemical and electrical gradients, a process called active transport (24). Absorption of enzymes is affected by several factors; a very important factor is the mixture containing the enzymes (6).
Licorice was chosen as the rich source of amylase enzyme for the following reasons:
1. it is a natural source of plant origin, and is used in Egypt as a popular drink after being fermented since hundreds or even thousands of years. To get a big amount of amylase in the prepared licorice, our preparation was concentrated 80 times that of the preparation prepared in the traditional way
2. it was discovered that licorice is very rich also in flavonoids (more than 50 substances) (1, 29) which are very important antioxidants and help to regain normal metabolism and treat diabetic complications. Recent researches confirmed that Flavonoids inhibit aldose reductase enzyme, thus preventing the conversion of excess glucose to sorbitol. The accumulation of sorbitol (in lens, retina, renal tissue, blood vessels, striated muscles, peripheral nerves, etc.) Is the cause of complications of diabetes mellitus (2, 3, 4, 28). Some researchers stated that inhibitors of aldose reductase would be the potent agents for the prevention of diabetic complications (27). Others stated that flavonoids are the most promising compound for the prevention and treatment of diabetic complications (26). Many experts showed that flavonoids in licorice had important therapeutic effects for the following complications of diabetes mellitus:
   A. Cataract (1).
   B. Peripheral neuritis (1).
   C. Nephropathy (1).
   D. Retinopathy (1).
      It is worth mentioning in that respect that there is a close relation, that has not been clarified before, between retinopathy and nephropathy. These two complications occur simultaneously after about 10-15 years from the beginning of the disease and gradually increase in intensity at almost the same rate. The appearance of one of them heralds the appearance of the other and nearly with the same intensity. Experimental work has proved that flavonoids present in licorice, as already mentioned , cure these two complications together by preventing the accumulation of sorbitol in renal and retinal tissues as well as in other tissues which are responsible for some other serious diabetic complications.
   E. Prevention of intra vascular clotting through anti-platelet aggregation (30, 31).
   F.Prevention of atherosclerosis: by several flavonoids in licorice, the most potent of which are polyphenolic flavonoids (32, 33, 34,35).
   G. Protection of hypercholesterolemia by preventing the peroxidation and damage of low density lipoprotein (32, 35). Thus preventing hypercholesterolemia which occurs as a result of destruction of low density lipoproteins. These lipoproteins are being destroyed by microphages due to their denaturation and peroxidation
   H.Reduction of plasma lipids levels (32)
   I. Decrease systolic blood pressure (32)
   J. Improving liver metabolism: this was manifested by inhibition of cytochrome oxidase p450 enzyme which becomes higher in diabetics than in normal person indicating the presence of impaired metabolism in the liver of diabetic patients (7,10). Flavonoids have been shown to be potent inhibitors of cytochrome enzyme p450 (7). It is worth mentioning that the improved metabolism in liver cells in diabetes by the use of flavonoids explains the success of these flavonoids in treating many important diabetic complications due to disturbed liver metabolism such as dislipidemia, hypercogulabilty atherosclerosis, coronary artery disease and others. Recent researches by hepatologists confirmed that these complications occur in diabetics with the beginning of appearance of disturbed liver metabolism starting as non-alcoholic steatohepatitis. Intensity of these complications increases with deterioration of the mitochondrial function in hepatocytes (12,14).

### How do flavonoids prevent diabetic complications in different body tissues?

Many researches have recently proved that flavonoids help in treatment and prevention of diabetic complications by preventing the accumulation of sorbitol in different body tissues. This is achieved by suppression of aldose reductase enzyme which initiates the first step in polyol pathway which occurs as a result of elevation of glucose concentration in tissues above normal levels which ends up in transformation of glucose into sorbitol (25,26,27,28).

### How do flavonoids regain normal vitality and functions of hepatocytes?

Flavonoids act as antioxidants. They regain normal enzymatic activity within hepatocytes by preventing accumulation of reactive oxygen species (ROS) that result as by- product of metabolic activity within the mitochondria (36). Inside theses mitochondria oxidative reduction reactions take place aiming at production of energy in form of Adenosine triphosphate (ATP) by the help of some important enzymes and coenzymes such as ATP synthase, cytochrome c oxidase, nicotinamide adenine dinucleotide (NAD) and flavine adenine dinucleotide (FAD). During these continuous enzymatic reactions small amount of reactive oxygen species (ROS) results which threatens to destroy cellular constituents. Some of NAD and FAD deal with these small amounts of ROS to protect cellular contents from destruction (16,23). The rest of NAD and FAD play their role in the process of normal metabolism and energy production inside cells. In diabetes mellitus, abnormal metabolism will result in accumulation of big amounts of ROS needing big amounts of NAD and FAD to deal with, with consequent deterioration in functions of NAD and FAD and their role in energy production and normal metabolism inside liver cells due to this abnormal pathway and abnormal consumption. Flavonoids deal and destroy excess ROS, thus sparing NAD and FAD to perform their normal activity in energy production and normal metabolism inside liver cells so liver regains its normal vitality and functions (36).

### Detailed Description

This drug is characterized by the following:
a. Mode of preparation of licorice in this drug differs from that of all preparations of licorice used for therapeutic purposes which deal with licorice root. This drug is prepared by fermentation of licorice for 6 hours (using water and sodium bicarbonate). The aim of fermentation is the production of large amounts of enzymes that treat with glucose. Root of licorice contains 30% starch, 2% glucose and 5% sucrose
b. Mode of exclusion of glycerizine in this drug differs from that used for getting deglycerizinized licorice (dgl) by boiling. here glycerizine is excluded by using diluted hydrochloric acid to protect the prepared enzymes from the effect of hyperthermia, as these enzymes may be damaged if exposed to temperature above 60c.

### The following materials are used for preparation of the drug:

1. Licorice root.
2. HCl (4 molar).
3. Sodium carbonate.

### Preparation of the drug in the form of syrup:

1. 250 gram of licorice root & 0.2 gram of sodium carbonate are added to 500 c.c. (cubic centimeters) of warm water between 20° C and 25°C.
   The mixture is left for 6 hours.
2. 750 ml of warm water between 20° C and 25° C is added to the mixture and left for 4 hours.
3. Diluted HCl is added then the mixture is stirred and filtered.
4. The filtrate is used as a drug. the dose is 5 ml (one tea spoonful) three times a day (TDS).

### Preparation of the drug in the form of capsules:

a- The drug is prepared in the form of soft gelatinous capsules according to the following steps:
   1- The solution is prepared according to the above mentioned steps.
   2- Reduction of water content is done by rotary suction pump.
   3- The produced dough is packed in soft gelatinous capsules. Each capsule contains a dose equivalent to 5ml of syrup form.
b- The drug is prepared in the form of hard capsules as follows:
   1- The solution is prepared according to the above mentioned steps.
   2- Dryness is done by lyophilization.
   3- Each capsule contains a dose equivalent to 5 ml syrup form.

**The effect of drug on some complications of diabetes in 62 patients**

| | **Complication** | **No. Of cases** | **Effect of the drug** |
|---|---|---|---|
| 1 | somnolence, the desire to relax or sleep for long hours | 23 | complete resolution within 3-4 wks in all cases. |
| 2 | fragility and easy fatigability | 24 | dramatic improvement in all cases within 3-4 wks. |
| 3 | shoulder pain and limitation of shoulder movement (up to frozen shoulder | 13 | complete resolution within 3-4 wks. |
| 4 | joint and bone aches in the hands, limbs, chest and toes, severe enough to keep patients awake all night. | 17 | pains relieved in less than 4 wks. in all cases |
| 5 | boils and skin infections (recurrent). | 14 | complete disappearance within 3-4 wks. |
| 6 | slow healing of wounds | 5 | resolved in all cases within 2-3 months |
| 7 | brittle finger nails | 7 | resumed their natural fitness within 2-3 months |
| 8 | hair falling off (heavily | 7 | hair regained its vividness and stopped falling off within 6-8 wks. |
| 9 | weak sensation in both feet | 13 | dramatic improvement in all cases within 3-4 wks. (some cases even resumed their "normal" sensation. |
| 10 | tingling and numbness in the limbs and the trunk. | 28 | resolved "completely" in 7 cases and improved remarkably in the remaining cases within 3-4 wks. |
| 11 | a sense of abnormal warmth in the sole of the feet. | 6 | resolved completely in all cases within 3-4 wks. |
| 12 | cramps of the limbs (severe enough to keep patients awake by night in some cases. | 17 | resolved completely in all cases within 3-4wks. |
| 13 | systemic primary hypertension | 32 | a-improved response to antihypertensive treatment in 5 cases (blood pressure range was 160-170/100-105-inspite of antihypertensive treatment) the pressure dropped to normal range with the use of the drug. b-significant reduction of the dose of antihypertensive drugs in 8 cases c- in one case: (case no .43) the blood pressure dropped to normal range (120/80) and antihypertensive were discontinued (the patient was resistant to antihypertensive before the use of the drug and the blood pressure was around 170/95 mmHg.) d- in the rest of cases, the blood pressure was almost normalized with the use of the usual dose of antihypertensive drugs along with the use of the drug. |
| 14 | weight loss (and even emaciation in some cases) inspite of normal appetite and normal food intake | 12 | patient resumed normal weight within 3-4 wks. |
| 15 | abnormal hyperphagia | 4 | patients regained normal appetite ( with the loss of the sense of continuous hunger ) within 2-3 wks |
| 16 | cataract | 1 | disappeared after one year of treatment (the diagnosis was confirmed by slit-lamp examination). |
| 17 | haziness and blurring of vision as a result of weak ocular muscles. | 5 | completely resolved in all cases within 3-4 wks. |
| 18 | weak body resistance presenting as upper respiratory tract infections recurring at remarkably short intervals and requiring antibiotic treatment | 6 | all cases significantly improved within 4-6 month as interpreted by the significant reduction of both the number and severity of attacks within that period. |
| 19 | amenorrhoea (in absence of pregnancy) in a 26 years old patient. | 1 | menstrual cycles recurred in a regular manner after two months of the use of the drug (after being lost for over a year). the patient got pregnant after 5 months of treatment. |
| 20 | bowel disturbances and severe constipation. | 5 | resolved completely in all cases within 3-4 wks. |
| 21 | diabetic foot with ulceration and impending gangrene | 1 | the condition improved dramatically with the use of the drug and the decision of below-knee amputation was ruled out within 3-4 wks. |
| 22 | fatty liver | 25 | only one case was followed up by repeated sonography. fatty liver disappeared after a few months and did not recur in the sonogram taken one year later |

### Comments on a few cases whose response with the use of the drug was dramatic:

### a-case 3:

a 20 years old patient could hardly tolerate walking for a few minutes or working over one hour after which she got extremely exhausted. This patient used to receive over 100 units of long acting insulin (mixtard) per day with a blood sugar level over 300 mg/dl rising frequently to deleterious levels requiring hospitalization. In short her life was miserable and she was severely depressed. With the use of the drug, this patient showed marked improvement within 2-3 wks. She could work for long hours and she was so happy about it that she walked to collage and back daily (over 5 kilometers) her insulin requirements dropped to around 25-30 u / day and her blood sugar dropped to acceptable levels (rarely exceeding 200 mg/ dl. Case 8 &16 showed an equivalent response.

### b- case 8:

A 45 yrs old male that was referred to in the previous section had a near frozen shoulder and was diagnosed as an advanced case of supra spinatous syndrome and was subjected to manipulation under anesthesia which failed to improve his condition within 3 wks of the use of the drug, this patient could move his shoulder painlessly in a more or less normal way.

### c- case 10:

This 40 years old male was emaciated and presented with multiple boils and carbuncles which required frequent surgical interference and that was incapacitating and practically put him out of work for long periods. all this disappeared after treatment with the drug for a few weeks.

### e-case 4:

This 60 years old patient suffered from complete loss of sensation in both soles and could not even feel his own shoes. This symptom lasted 3 yrs. In less than 4 wks of treatment he recovered a good deal of his sensation.

### Mode of application:

This drug is used for treatment of complications of diabetes mellitus.

### Materials used for preparation of the drug::

1- Licorice root.
2- HCl (4 molar).
3- Sodium carbonate

### Preparation of the drug in the form of syrup:

1- 250 gram of licorice root & 0.2 gram of sodium carbonate are added to 500 c. c. (cubic centimeters) of warm water between 20° C and 25° C. The mixture is left for 6 hours.
2- 750 ml of warm water between 20° C and 25° C is added to the mixture and left for 4 hours.
3- Diluted HCl is added then the mixture is stirred and filtered.
4- The filtrate is used as a drug. The dose is 5 ml (one tea spoonful) three times a day (TDS).

### Preparation of the drug in the form of capsules:

a- The drug is prepared in the form of soft gelatinous capsules according to the following steps:
   1- The solution is prepared according to the above mentioned steps.
   2- Reduction of water content is done by rotary suction pump.
   3- The produced dough is packed in soft gelatinous capsules. each capsule contains a dose equivalent to 5ml of syrup form.
b- The drug is prepared in the form of hard capsules as follows:
   1- The solution is prepared according to the above mentioned steps.
   2- Dryness is done by lyophilization.
   3- Each capsule contains a dose equivalent to 5 ml syrup form.

In further special embodiments the method of preparing a drug for treatment of diabetes mellitus complications is characterized in that the mixture of licorice, sodium carbonate and water (step 1) is kept for a period ranging from 2 to 8 hours, preferably from 4 to 6 hours and most preferably for 6 hours.

In another special embodiment the method of preparing a drug for treatment of diabetes mellitus complications is characterized in that the mixture of step 2 is kept for a period ranging from 2 to 6 hours, preferably from 3 to 5 hours and most preferably for 4 hours.

In yet another special embodiment the medicament formulation consists of licorice root, sodium carbonate, HCl and water after fermentation for a period ranging from 6 to 16 hours, preferably from 8 to 14 hours and most preferably for 12 hours.

### References

1. Carol A. Newall, Linda A. Anderson and David J. (1996): Herbal medicines, a guide for health care professionals. London, the pharmaceutical press.
2. Aida K.E. et al. (1990): Isoliquiritigenin: A New Aldose Reductase Inhibitor From Glycyrrhizae Radix Planta Med, 56: 254-8.
3. Yun-Ping Z., Jia -Qing Z. (1989): Oral baicalin and liquid extract of licorice reduce sorbitol levels in red blood cells of diabetic rats. Chin Med J, 102: 203-6.
4. Inque H. et al. (1987): Pharmacological activities of glycerrhetinic acid derivatives: analgesic and anti - type allergic effects. Chem Pharm Bull, 35:3888-93.
5. Ganong W. (1993): Review of medical physiology, 16th ( edit,) prentice - hall international inc. (pub), (19); 310:
6. Lopez D.A., Williams R,M and Miehlke S. ( 1994): Enzymes the fountain of life, 1 st ( edit ), the neville press inc. (pub ). Munchen, 34-37.
7. Hamdy A.M. (2000): Biochemical study on the effects of some Egyptian herbs in alloxan - induced diabetic rats, toxicology 174, 131-139.
8. Rosenblum J.L., Raab B.K. and Alpers D.H. (1982): Hepatobiliary and pancreatic clearance of circulating pancreatic amylase Am J Physoil., Jul; 243 (1):21-7.
9. Leibow C. and Rothman S.S. (1975): Enteropancreatic circulation of digestive enzymes. Science 8; 189 (4201): 472-4.
10. Dessayre D., Berson A., Fromenty B. and Mansouri A. ( 2001 ): Mitochondrial Dysfunction In Hepatic Steatosis In Leuschner U, James O And Dancygier H ( Edit ) Falk Symposium 121 Steatohepatitis ( Nash And Ash ), Kluwer Academic Publishers ( Pub ), London; 80-100.
11. Koyqma I.K. and Hokari S. (2001): Expression of alpha amylase gene in rat liver: liver - specific amylase has a high affinity to glycogen. Electrophoresis; 22 (1) : 12-7.
12. Lonardo A., Loria P. and Carulli M. (2001): Insulin Resistance In Non - Alcoholic Fatty Liver Disease : A Clinical Perspective In Leuschner U, James O And Dancygier H (Edit ) Falk Symposium 121 Steatohepatitis (Nash And Ash), Kluwer Academic Publishers (Pub ), London; 104-113.
13. Niesen T.E., Alpres D.H., Rosenblum J.L. (1984): Metabolism of glycosylated human salivary amylase in vivo plasma clearance by rat hepatic endothelial cells and in vitro receptor mediated pinocytosis by rat macrophages. J Leukoc Boil; 36 (3) : 307-20.
14. **Sanyal AJ, Campbell - Sargent C , Mirshahi F, Rizzo WB , Contos MJ and Clore J (2001):** The intrahepatic and extrahepatic metabolic abnormalities associated with non- alcoholic steatohepatitis. In leuschner U, James O and Dancygier H (edit ) Falk symposium 121 Steatohepatitis ( NASH and ASH) , Kluwer Academic Publishers (Pub) London ; 172-81.
15. Schalm S.W. and Von Hoek B. (2001): Treatment of non- alcoholic steatohepatitis role of ursodeoxycholic acid. In Ieuschner U, James O and Dancygier H( edit) Falk symposium 121 Steatohepatitis ( NASH and ASH ) Kluwer Academic Publishers (Pub), London; 185-187.
16. Fromenty B., Degoul F., Demeilliers C., Sutton A., Mansouri A. and Pessayre D. (2001): Alcohol and mitochondrial function in Ieuschner U, James O and Dancygier H (edit ) Falk Symposium 121 Steatohepatitis ( NASH and ASH) Kluwer Academic Publishers ( Pub) London ; 257- 275.
17. Matthews D.M. (1992): Protein absorption. Wiley- Liss, New York.
18. Siamak A.A. (1997): The oligopeptide transporter (pept-1-) in human intestine: biology and function. Gastroenterology; 113:332 -340.
19. Curr Drug Metab (2004); 5 (1) : 85 -94
20. Sterling M.R. (1999): Can cirrhosis be prevented ? , Issue of Nutrition Science News Jan.
21. Tom Brody (2001): Anti oxidants. in nutritional biochemistry, 2nd edition. Academic Press.
22. Dufour J.F. (2001): Anti oxidants in the treatment of non alcoholic steatohepatitis. In Leuschner U, James O and Dancygier H (edit ) Falk symposium 121 Steatohepatitis (NASH and ASH), Kluwer Academic Publishers (Pub) London ; 197-202.
23. Balz F. (1994): Reactive oxygen species and antioxidant vitamins. American journal of medicine, vol. 97, page 3a- 7s, by Excerpta Medica.
24. Phillip P.T. (1996): Malabsorption. in Claude Bennett and Fred Plum, Cecil Textbook of Medicine, 20th edition. W.B. Saunders company.
25. Zhou Y., Zhang J. (1990): Effects of baicalin and liquid extract of licorice on sorbitol level in red blood cells of diabetic rats -Zhongguo Zhong Yao Zazhi.; 15 (7): 433-5, 448.
26. Lim S.S., Jung S.H., Ji J., Shin K.H., Keum S.R. (2001): Synthesis of flavonoids and their effects on aldose reductase and sorbitol accumulation in streptozotocin - induced diabetic at tissues. Pharmacol.;53(5): 653-68.
27.Jung S.H., Lee Y.S., Shim S.H. (2005): Inhibitory effects of Ganoderma applanatum on rat lens aldose reductase and sorbitol accumulation in streptozotocin - induced diabetic rat tissues. Phytother Res.; 19(6): 477-80.
28. Cameron N.E., Cotter M.A., Basso M. (1997): Comparison of the effects of inhibitors of aldose reductase and sorbitol dehydrogenase on neurovascular function, nerve conduction and tissue polyol pathway metabolites in streptozotocin - diabetic rats. Diabetologia.; 40(3): 271-81.
29. Kitagawa L., Chen W.Z., Taniyama T., Harada E., Hori K., Kobayashi M., Ren J. (1998): Quantitative determination of constituents in various licorice roots by means of high performance liquid chromatography. Yakugaku Zasshi.; 118( 11) : 519-28.
30.Zhan C., Yang J. (2006): Protective effects of isoliquiritigenin in transient middle cerebral artery occlusion - induced focal cerebral ischemia in rats. Pharmacol Res.; 53 (3): 303 -9.
31. Tawata M., Aida K., Noguchi T., Ozaki Y., Kume S., Sasaki H., Chin M., Onaya T. (1992): Anti- platelet action of isoliquiritigenin an aldose reductase inhibitor in licorice. Eur J Pharmacol, 25; 212 (1) : 87-92.
32. Fuhrman B., Volkova N., Kaplan M., Presser D., Attias J., Hayek T., Aviram M. (2002): Anti atherosclerotic effects of licorice extract supplementation on hypercholesterolemic patients. Nutrition.; 18(3) : 268-73.
33. Aviram M., Fuhrman B. (1998): Polyphenolic flavonoids inhibit microphage - mediated oxidation of LDL and attenuate atherogenesis. Atherosclerosis. 137 Suppl : 45- 50.
34. Belinky P.A., Aviram M., Fuhrman B., Rosenblat M., Vaya J. (1998): The antioxidative effects of the isoflavan glabridin on endogenous constituents of LDL during its oxidation atherosclerosis.; 137(1) : 49-61.
35.Fuhrman B., Buch S., Vaya J., Belinky P.A., Coleman R., Hayek T., Avirm M. (1997): Licorice extract and its major polyphenol glabridin protect low - density lipoprotein against lipid peroxidation: in vitro and ex vivo studies in humans and in atherosclerotic apolipoprotein E- deficient mice. Am J Clin Nutr.; 66(2): 267-75
36. Haraguchi H., Yoshida N., Ishikawa H.(2000): Protection of mitochondrial functions againts oxidative stresses by isoflavans from glycyrhiza glabra. J Pharm Pharmacol.; 52(2) 219-23.

## Claims

1. Method of preparing a drug for treatment of diabetes mellitus complications comprising:
1- 250 gram of licorice root & 0.2 gram of sodium carbonate are added to 500 c. c. (cubic centimeters) of warm water.
2- 750 ml of warm water is added to the mixture.
3- Diluted HCl is added then the mixture is stirred and filtered.

2. Method according to claim 1 **characterized in that** the mixture of licorice, sodium carbonate and water ( step 1 ) is kept for a period ranging from 2-8 hours.

3. Method according to one of the preceding claims **characterized in that** the mixture of licorice, sodium carbonate and water ( step 1 ) is kept for a period ranging from 4 to 6 hours.

4. Method according to claim 1 **characterized in that** the mixture of step 2 is kept for a period ranging from 2-6 hours.

5. Method according to claims 1 or 4 **characterized in that** the mixture of step 2 is kept for a period ranging from 3 to 5 hours.

6. Method according to one of the preceding claims, **characterized in that** the drug is concentrated by a rotary suction pump or lyophilized and packed in soft gelatinous capsules or hard capsules respectively,

7. Medicament formulation consisting of licorice root, sodium carbonate, HCl and water after fermentation for a period ranging from 6 to 16 hours.

8. Medicament formulation consisting of licorice root, sodium carbonate, HCl and water after fermentation for a period ranging from 8 to 14 hours.

## Patentansprüche

1. Verfahren zur Herstellung eines Arzneimittels zur Behandlung von Diabetes Mellitus Komplikationen, umfassend:
1- 250 Gramm Süßholzwurzel und 0,2 Gramm Natriumcarbonat werden in 500 cm³ (Kubikzentimer) warmes Wasser gegeben.
2- 750 ml warmes Wasser wird der Mischung beigegeben.
3- verdünntes HCl wird hinzugefügt, dann wird die Mischung gerührt und gefiltert.

2. Verfahren nach Anspruch 1, **dadurch gekennzeichnet, dass** die Mischung aus Süßholz, Natriumcarbonat und Wasser (Schritt 1) über einen Zeitraum von 2 bis 8 Stunden aufrechterhalten wird.

3. Verfahren nach einem der vorstehenden Ansprüche, **dadurch gekennzeichnet, dass** die Mischung aus Süßholz, Natriumcarbonat und Wasser (Schritt 1) über einen Zeitraum von 4 bis 6 Stunden aufrechterhalten wird.

4. Verfahren nach Anspruch 1, **dadurch gekennzeichnet, dass** die in Schritt 2 entstandene Mischung über einen Zeitraum von 2 bis 6 Stunden aufrechterhalten wird.

5. Verfahren nach den Ansprüchen 1 oder 4, **dadurch gekennzeichnet, dass** die in Schritt 2 entstandene Mischung über einen Zeitraum von 3 bis 5 Stunden aufrechterhalten wird.

6. Verfahren nach einem der vorstehenden Ansprüche, **dadurch gekennzeichnet, dass** das Arzneimittel durch eine Rotationssaugpumpe konzentriert oder lyophilisiert wird und in weichen gelartigen Kapseln bzw. in harten Kapseln abgefüllt wird.

7. Arzneimittel nach einer Formel bestehend aus Süßholzwurzel, Natriumcarbonat, HCl und Wasser nach Gärung über einen Zeitraum von 6 bis 16 Stunden.

8. Arzneimittel nach einer Formel bestehend aus Süßholzwurzel, Natriumcarbonat, HCl und Wasser nach Gärung über einen Zeitraum von 8 bis 14 Stunden.

## Revendications

1. Procédé de préparation d'un médicament pour le traitement de complications du diabète, comprenant :
1-250 grammes de racine de réglisse et 0,2 grammes de carbonate de sodium sont ajoutés à 500 cm³ (centimètres cube) d'eau chaude.
2-750 ml d'eau chaude sont ajoutés au mélange.
3- du HCl dilué est rajouté, puis on remue le mélange et on le filtre.

2. Procédé selon la revendication 1, **caractérisé en ce que** le mélange de réglisse, de carbonate de sodium et d'eau (étape 1) est maintenu pendant une période comprise entre 2 et 8 heures.

3. Procédé selon l'une quelconque des revendications précédentes, **caractérisé en ce que** le mélange de réglisse, de carbonate de sodium et d'eau (étape 1) est maintenu pendant une période comprise entre 4 et 6 heures.

4. Procédé selon la revendication 1, **caractérisé en ce que** le mélange tel qu'il a été réalisé à l'étape 2 est maintenu pendant une période comprise entre 2 et 6 heures.

5. Procédé selon les revendications 1 ou 4, **caractérisé en ce que** le mélange tel qu'il a été réalisé à l'étape 2 est maintenu pendant une période comprise entre 3 et 5 heures.

6. Procédé selon l'une quelconque des revendications précédentes, **caractérisé en ce que** le médicament est concentré par une pompe aspirante rotative ou lyophilisé et conditionné dans des capsules molles gélatineuses ou dans des capsules dures.

7. Formulation d'un médicament constituée de racine de réglisse, de carbonate de sodium, de HCl et d'eau après fermentation pendant une période comprise entre 6 et 16 heures.

8. Formulation d'un médicament constituée de racine de réglisse, de carbonate de sodium, de HCl et d'eau après fermentation pendant une période comprise entre 8 et 14 heures.
